# EUROPEAN PATENT APPLICATION

(11) **EP 1 553 073 A1**
(43) Date of publication of application: **13.07.2005**
(21) Application number: 03733412.5
(22) Date of filing: 13.06.2003
(51) Int. Cl.: C07C 29/147, C07C 31/22, C07M 7/00, C07B 53/00

(54) **PROCESS FOR PRODUCTION OF 1,2,4-BUTANETRIOL**

(30) Priority: 14.06.2002 JP 2002173625
(71) Applicant: DAISO CO., LTD., Osaka-shi, Osaka 550-0002 (JP)
(72) Inventor: FURUKAWA, Yoshiro, c/o DAISO CO., LTD, Osaka-shi, Osaka 550-0002 (JP); OHTAKA, Seiji, c/o DAISO CO., LTD, Osaka-shi, Osaka 550-0002 (JP)
(74) Representative: Peaucelle, Chantal
(86) International application number: PCT/JP2003/007530
(87) International publication number: WO 2003/106392

(57) **Abstract**

An object of the present invention is to provide a simple and low-cost process for preparing 1,2,4-butanetriol. Malic diester, 3-hydroxy-γ-butyrolactone or 3,4-dihydroxybutanoate represented by the following formula (I) or a mixture thereof, which is a raw material, is reduced in a medium in the presence of sodium borohydride to give 1,2,4-butanetriol represented by the following formula (II). In the process, sodium borohydride is added to a first alcohol, the raw material is added to a second alcohol which is different from the first alcohol, and they are reacted each other. In the formula, R is alkyl having four or less carbon atoms.

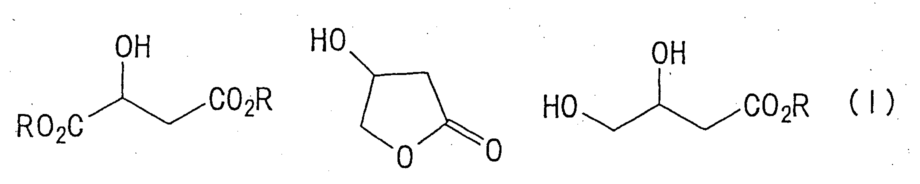

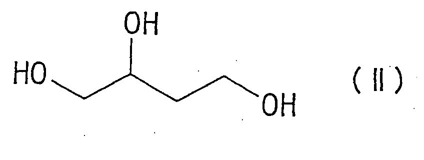

## Description

### Technical Field

The present invention relates to a process for preparing 1,2,4-butanetriol, which is a useful compound as a synthetic intermediate of pharmaceuticals, agricultural chemicals and the like.

### Background Art

1,2,4-Butanetriol is an industrially useful compound which is known from old times, and it is widely used as a synthetic precursor of pharmaceuticals. A process for synthesizing the compound is already known by WO99/44976, but the target product cannot be obtained in a high yield though a relatively large amount of sodium borohydride is used as a reducing agent in the process.

An object of the present invention is to provide a simple and low-cost process for preparing 1,2,4-butanetriol.

Studying intensively in order to achieve the above-mentioned object, the present inventors found a process which can improve the amount of sodium borohydride to be used and the yield of the product.

### Disclosure of the Invention

A process for preparing 1,2,4-butanetriol according to the present invention is a process wherein malic diester, 3-hydroxy-γ-butyrolactone or 3,4-dihydroxybutanoate represented by the following formula (I) or a mixture thereof, which is a raw material, is reduced in a medium in the present of sodium borohydride to give 1,2,4-butanetriol represented by the following formula (II), characterized in that sodium borohydride is added to a first alcohol, the raw material is added to a second alcohol which is different from the first alcohol, and then they are subjected to the reduction, or the raw material is added to a mixed solvent of the first alcohol and the second alcohol which is different from the first alcohol, and sodium borohydride is added thereto to reduce the raw material. 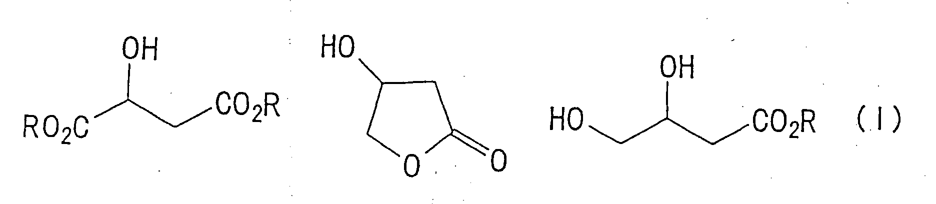 (wherein R is alkyl having four or less carbon atoms)

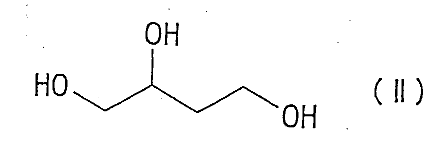

A first reaction mode of the process of the present invention is characterized in that to the first alcohol containing sodium borohydride is added a solution containing malic diester, 3-hydroxy-γ-butyrolactone or 3,4-dihydroxybutanoate represented by the above formula (I) or the mixture thereof, which is a raw material, in the second alcohol which is different from the first alcohol to reduce the raw material to give 1,2,4-butanetriol represented by the above formula (II).

A second reaction mode of the process of the present invention is characterized in that the first alcohol containing sodium borohydride is added to a solution containing malic diester, 3-hydroxy-γ-butyrolactone or 3,4-dihydroxybutanoate represented by the above formula (I) or the mixture thereof, which is a raw material, in the second alcohol which is different from the first alcohol to reduce the raw material to give 1,2,4-butanetriol represented by the above formula (II).

A third reaction mode of the process of the present invention is characterized in that malic diester, 3-hydroxy-γ-butyrolactone or 3,4-dihydroxybutanoate represented by the above formula (I) or a mixture thereof, which is a raw material, is added to a mixed solvent of a first alcohol and a second alcohol which is different from the first alcohol, and then sodium borohydride is added thereto to reduce the raw material to give 1,2,4-butanetriol represented by the above formula (II),

When optically active malic diester, optically active 3-hydroxy-γ-butyrolactone or optically active 3,4-dihydroxybutanoate represented by the following formula (III) is used as the raw material in the process of the present invention, optically active 1,2,4-butanetriol represented by the following formula (IV) is obtained by reduction. 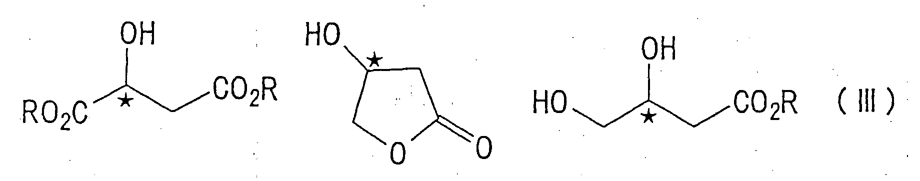 (wherein R is alkyl having four or less carbon atoms)

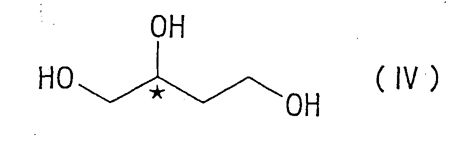

Malic diester, 3-hydroxy-γ-butyrolactone or 3,4-dihydroxybutanoate is used as the raw material of the process of the present invention. The alkyl group R of malic diester and 3,4-dihydroxybutanoate is not limited so far as it has four or less carbon atoms, but methyl or ethyl is preferable in terms of reactivity of reduction. Thus, when the optically active substance is used as the raw material, optically active 1,2,4-butanetriol is obtained by reduction.

Two groups of alcohols which are different each other are used as the reaction media. They are referred to as "the first alcohol" and "the second alcohol" throughout the present Description and Claims. When sodium borohydride is added to the first alcohol, sodium borohydride is hardly dissolved in the first alcohol, and at least a portion of it, substantially the whole, is suspended in the first alcohol. When the raw material is added to the second alcohol, the raw material is dissolved in the second alcohol.

The first alcohol is alcohol having preferably four or more carbon atoms, more preferably four to ten carbon atoms such as 1-butanol, 2-butanol, 2-methyl-2-propanol or a mixture thereof. The second alcohol is preferably alcohol having three or less carbon atoms, more preferably methanol, ethanol or a mixture thereof.

An amount of the first alcohol is preferably three, to ten-fold, more preferably five to seven-fold expressed in gram ratio to sodium borohydride. An amount of the second alcohol is preferably 0.3 to 1.5-fold, more preferably 0.5 to 0.9-fold expressed in gram ratio to the raw material.

There are three reaction modes. The first reaction mode is characterized in that the solution containing the raw material in the second alcohol is added to the first alcohol containing sodium borohydride. The second reaction mode is characterized in that the first alcohol containing sodium borohydride is added to the solution containing the raw material in the second alcohol. The third reaction mode is characterized in that sodium borohydride, as it is, is added to a mixed solvent containing the raw material, and the mixed solvent comprises the first alcohol and the second alcohol which is different from the first alcohol.

Incidentally, a reaction was attempted using an ethereal solvent, specifically tetrahydrofuran (THF) instead of the first alcohol as a solvent which suspends a portion of sodium borohydride or substantially the whole. As a result, it turned out that the yield of the product is at most about 80% in this case. Further, it is feared that THF is easily oxidized in air to give a colorless explosive peroxide as a general property. Accordingly, it is not preferable to use THF as a solvent for suspending sodium borohydride.

An amount of sodium borohydride in the process of the present invention is preferably one to five equivalents, more preferably 1.5 to 1.8 equivalents expressed in molar ratio to the raw material. Two or more equivalents of sodium borohydride are used to the raw material in Examples of WO99/44976. Accordingly, it is apparent that the present invention enables the amount of sodium borohydride to be reduced. When the amount of sodium borohydride is too small, the reaction is not completed, and the yield is apt to be lowered. To the contrary, when it is used in excess, a production cost is raised, and a large amount of a salt is formed and thereby later filtration procedure is difficult. Accordingly, such amounts are not preferable.

A reaction temperature in the process of the present invention is preferably - 20° to 80°C , more preferably 0° to 50°C during addition, and the temperature is preferably 30° to 80°C , more preferably 50° to 70°C during maturation after addition. The reaction is usually carried out at an ordinary pressure and can be optionally carried out under an elevated pressure. Reaction time is adjusted depending on the reaction temperature and the reaction pressure.

### Best Mode for Carrying out the Invention

The present invention is practically described below by Examples, but the present invention is not limited to these Examples.

### Example 1: Preparation of (S)-1,2,4-butanetriol

i) Sodium borohydride (41.5 g, 1.1 mol) was added to tert-butanol (260 g) to suspend it. To the obtained suspension was added a solution of dimethyl (S)-malate (100 g, 0.617 mol) in methanol (78 g) dropwise.
ii) The obtained mixture was stirred at 70°C for five hours, a 35% aqueous HCl solution (120 ml) was added to the reaction mixture, and the resulting insoluble matter was filtered out. Next, methanol (1,600 ml) was added to the filtrate, and methanol was distilled away under an acidic condition to remove boron. Then the residue was purified by distillation under a reduced pressure to give 59.2 g (0.558 mol) of (S)-1,2,4-butanetriol. Yield: 90.4%, optical purity: 99.1%ee.

### Example 2: Preparation of (S)-1,2,4-butanetriol

The mixture obtained in the same manner as in the step i) of Example 1 was stirred at 70°C for five minutes, and a 35% aqueous HCl solution (120 ml) was added to the reaction mixture, followed by filtering out the resulting insoluble matter. The obtained filtrate was passed through a column packed with an ion exchange resin ("XE-583" manufactured by Organo Co., Ltd., 200 g) to remove remaining boron and then concentrated under reduced a pressure. Further, the residue was purified by distillation to give 58.6 g (0.552 mol) of (S)-1,2,4-butanetriol. Yield: 89.5%, optical purity: 99.0%ee.

### Example 3: Preparation of (S)-1,2,4-butanetriol

Sodium borohydride (41.5 g, 1.1 mol) was added to sec-butanol to suspend it. To the obtained suspension was added a solution of dimethyl (S)-malate (100 g, 0.617 mol) in methanol (78 g) dropwise.

Next, the same procedure as in the step ii) of Example 1 was repeated to give 58.3 g (0.549 mol) of (S)-1,2,4-butanetriol. Yield: 89.0%, optical purity: 99.3%ee.

### Example 4: Preparation of (S)-1,2,4-butanetriol

A suspension of sodium borohydride (41.5 g, 1.1 mol) in see-butanol (260 g) was added dropwise to a solution of dimethyl (S)-malate (100 g, 0.617 mol) in methanol (78 g).

Next, the same procedure as in the step ii) of Example 1 was repeated to give 58.6 g (0.552 mol) of (S)-1,2,4-butanetriol. Yield: 89.5%, optical purity: 99.0%ee.

### Example 5: Preparation of (S)-1,2,4-butanetriol

A suspension of sodium borohydride (41.5 g, 1.1 mol) in tert-butanol (260 g) was added dropwise to a solution of dimethyl (S)-malate (100 g, 0.617 mol) in methanol (78 g).

Next, the same procedure as in the step ii) of Example 1 was repeated to give 58.9 g (0.555 mol) of (S)-1,2,4-butanetriol. Yield: 90.0%, optical purity: 99.0%ee.

### Example 6: Preparation of (S)-1,2,4-butanetriol

i) Sodium borohydride (42.0 g, 1.1 mol) was gradually added to a solution of dimethyl (S)-malate (100 g, 0.617 mol) in a mixed solvent of tert-butanol (400 g) and methanol (90 g).
ii) The mixture was stirred at 40°C for two hours, and a 35% aqueous HCl solution (142 g) was added to the reaction mixture, followed by filtering out the resulting insoluble matter. Next, methanol (1,600 ml) was added to the filtrate, and methanol was distilled away under an acidic condition to remove boron. Then the residue was purified by distillation under a reduced pressure to give 60.9 g (0.574 mol) of (S)-1,2,4-butanetriol. Yield: 93.0%, optical purity: 99.0%ee.

### Comparative Example 1: Use of only ethanol as a solvent

i) Sodium borohydride (41.5 g, 1.1 mol) was added to a solution of dimethyl (S)-malate (100 g, 0.617 mol) in ethanol (260 g) while keeping the temperature at 20°C.
ii) The mixture was stirred at 20°C for 15 hours, then the reaction mixture was cooled, and saturated HC1-EtOH (200 ml) was added thereto, followed by filtering out the resulting insoluble matter. The filtrate was passed through a column packed with an ion exchange resin ("XE-583" manufactured by Organo Co., Ltd., 200 g) to remove remaining boron and then concentrated under a reduced pressure. Further, the residue was purified by distillation to give 42.6 g (0.401 mol) of (S)-1,2,4-butanetriol. Yield: 65.0%, optical purity: 99.0%ee.

### Comparative Example 2: Use of toluene and ethanol as solvents

i) Sodium borohydride (41.5 g, 1.1 mol) was added to toluene (260 g), and a solution of dimethyl (S)-malate (100 g, 0.617 mol) in methanol (78 g) was added dropwise thereto.
ii) The obtained mixture was stirred at 20°C for six hours, then the reaction mixture was cooled, and saturated HCl-EtOH (200 ml) was added thereto, followed by filtering out the resulting insoluble matter. The filtrate was passed through a column packed with an ion exchange resin ("XE-583" manufactured by Organo Co., Ltd., 200 g) to remove remaining boron and then concentrated under a reduced pressure. Further, the residue was purified by distillation to give 45.9 g (0.433 mol) of (S)-1,2,4-butanetriol. Yield: 70.2%, optical purity: 99.0%ee.

### Industrial Applicability

Since sodium borohydride is added to a first alcohol, a raw material is added to a second alcohol which is different from the first alcohol, and then they are reacted each other according to the present invention, sodium borohydride is suspended in the first alcohol and participates in the reaction at a high concentration, and its utilization efficiency is high. Accordingly, the reaction proceeds more efficiently, compared with a case where one alcohol is used as a reaction medium and a case where sodium borohydride is suspended in toluene and the raw material is dissolved in methanol. Consequently, the yield of the product is remarkably improved even if an amount of expensive sodium borohydride is reduced, and 1,2,4-butanetriol is prepared inexpensively.

In a reaction mode wherein the raw material is added to a mixed solvent of the first alcohol and the second alcohol which is different from the first alcohol and sodium borohydride is added thereto, the yield of the product is much more increased, and the reaction time is shortened compared with the above-mentioned reaction mode wherein sodium borohydride is added to the first alcohol.

## Claims

1. A process for preparing 1,2,4-butanetriol wherein malic diester, 3-hydroxy-γ-butyrolactone or 3,4-dihydroxybutanoate represented by the following formula (I) or a mixture thereof, which is a raw material, is reduced in a medium in the present of sodium borohydride to give 1,2,4-butanetriol represented by the following formula (II), **characterized in that** sodium borohydride is added to a first alcohol, the raw material is added to a second alcohol which is different from the first alcohol, and then they are subjected to the reduction, 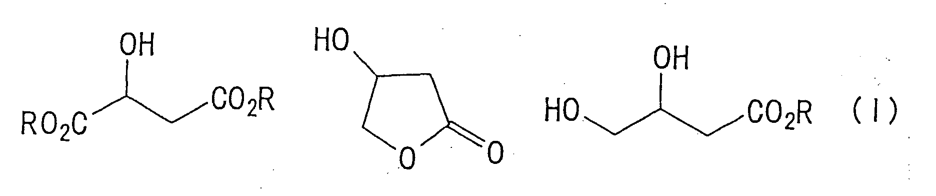 wherein R is alkyl having four or less carbon atoms.

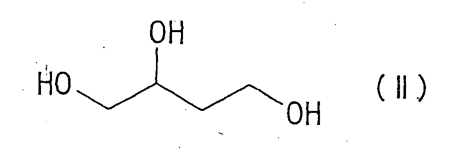

2. A process for preparing 1,2,4-butanetriol **characterized in that** to a first alcohol containing sodium borohydride is added a solution containing malic diester, 3-hydroxy-γ-butyrolactone or 3,4-dihydroxybutanoate represented by the following formula (I) or a mixture thereof, which is a raw material, in a second alcohol which is different from the first alcohol to reduce the raw material to give 1,2,4-butanetriol represented by the following formula (II), 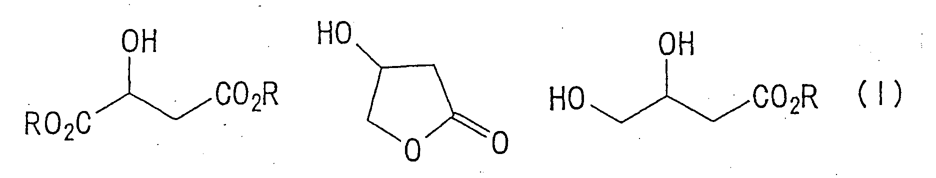 wherein R is alkyl having four or less carbon atoms.

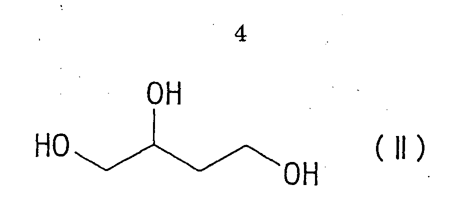

3. A process for preparing 1,2,4-butanetriol **characterized in that** a first alcohol containing sodium borohydride is added to a solution containing malic diester, 3-hydroxy-γ-butyrolactone or 3,4-dihydroxybutanoate represented by the following formula (I) or a mixture thereof, which is a raw material, in a second alcohol which is different from the first alcohol to reduce the raw material to give 1,2,4-butanetriol represented by the following formula (II), 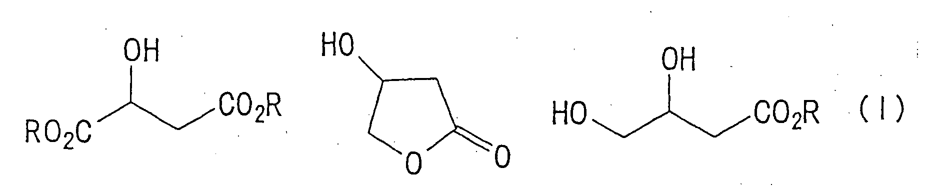 wherein R is alkyl having four or less carbon atoms.

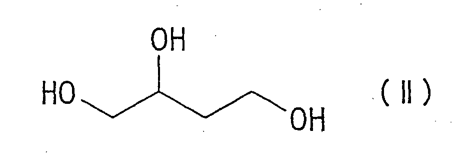

4. A process for preparing 1,2,4-butanetriol **characterized in that** malic diester, 3-hydroxy-γ-butyrolactone or 3,4-dihydroxybutanoate represented by the following formula (I) or a mixture thereof, which is a raw material, is added to a mixed solvent of a first alcohol and a second alcohol which is different from the first alcohol, and then sodium borohydride is added thereto to reduce the raw material to give 1,2,4-butanetriol represented by the following formula (II), 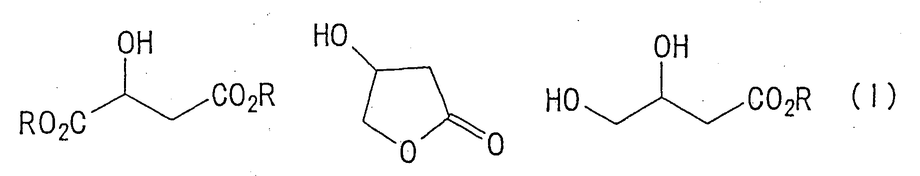 wherein R is alkyl having four or less carbon atoms.

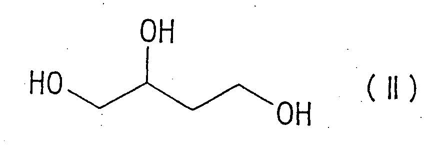

5. The process for preparing 1,2,4-butanetriol as claimed in any one of claims 1 to 4, **characterized in that** the raw material is optically active malic diester, optically active 3-hydroxy-γ-butyrolactone or optically active 3,4-dihydroxybutanoate represented by the following formula (III), and the compound obtained by reduction is optically active 1,2,4-butanetriol represented by the following formula (IV), 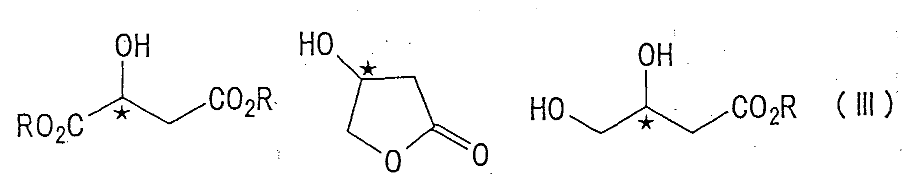 wherein R is alkyl having four or less carbon atoms.

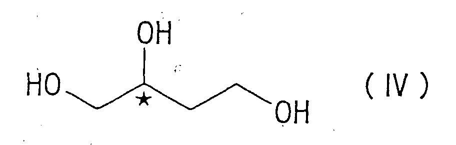

6. The process for preparing 1,2,4-butanetriol as claimed in any one of claims 1 to 3 and 5, **characterized in that** at least a portion of sodium borohydride is suspended in the first alcohol.

7. The process for preparing 1,2,4-butanetriol as claimed in any one of claims 1 to 6, **characterized in that** the first alcohol is an alcohol having four or more carbon atoms, and the second alcohol is an alcohol having three or less carbon atoms.

8. The process for preparing 1,2,4-butanetriol as claimed in claim 7, **characterized in that** the first alcohol is 1-butanol, 2-butanol, 2-methyl-2-propanol or a mixture thereof, and the second alcohol is methanol, ethanol or a mixture thereof.
